# EUROPEAN PATENT SPECIFICATION

(11) **EP 3 463 357 B1**
(45) Date of publication and mention of the grant of the patent: **20.09.2023**
(21) Application number: 17729263.8
(22) Date of filing: 23.05.2017
(51) Int. Cl.: A61K 31/454, A61P 25/22

(54) **(2S)-1-[4-(3,4-DICHLOROPHENYL)PIPERIDIN-L-YL]-3-[2-(5-METHYL-L,3,4-OXADIAZOL-2-YL)BENZO[B]FURAN-4-YLOXY]PROPAN-2-OL OR ITS METABOLITE FOR TREATING ANXIETY DISORDERS**
(2S)-1-[4-(3,4-DICHLORPHENYL)PIPERIDIN-L-YL]-3-[2-(5-METHYL-L,3,4-OXADIAZOL-2-YL)BENZO[B]FURAN-4-YLOXY]PROPAN-2-OL ODER METABOLIT DAVON ZUR BEHANDLUNG VON ANGSTZUSTÄNDEN
(2S)-1-[4-(3,4-DICHLOROPHÉNYL)PIPÉRIDINE-L-YL]-3-[2-(5-MÉTHYL-L,3,4-OXADIAZOL-2-YL)BENZO[B]FURANE-4-YLOXY]PROPANE-2-OL OU SON MÉTABOLITE POUR LE TRAITEMENT DE TROUBLES ANXIEUX

(30) Priority: 25.05.2016 US 201662341517 P; 11.08.2016 US 201662373720 P
(43) Date of publication of application: 10.04.2019
(73) Proprietor: Minerva Neurosciences, Inc., Waltham, MA 02451 (US)
(72) Inventor: LUTHRINGER, Remy, 1204 Geneva (CH)
(74) Representative: Cooley (UK) LLP
(86) International application number: PCT/US2017/034036
(87) International publication number: WO 2017/205399

(56) References cited:
- US-A1- 2002 111 358
- US-A1- 2014 206 722
- PAPP M ET AL: "P.2.b.008 Antidepressant-like activity of Wf-516, an antagonist of 5-HT1A receptors and inhibitor of 5-HT reuptake, in a chronic mild stress model of depression in rat", EUROPEAN NEUROPSYCHOPHARMACOLOGY, ELSEVIER SCIENCE PUBLISHERS BV, AMSTERDAM, NL, vol. 16, 1 January 2006 (2006-01-01), pages S305-S306, XP025110301, ISSN: 0924-977X, DOI: 10.1016/S0924-977X(06)70322-4 [retrieved on 2006-01-01]
- GRIEBEL G ET AL: "The effects of compounds varying in selectivity as 5-HT"1"A receptor antagonists in three rat models of anxiety", NEUROPHARMACOLOGY, PERGAMON PRESS, OXFORD, GB, vol. 39, no. 10, 1 September 2000 (2000-09-01), pages 1848-1857, XP027251972, ISSN: 0028-3908 [retrieved on 2000-09-01]

## Description

### RELATED APPLICATIONS

This application claims priority to, and the benefit of, U.S. Provisional Application Nos. 62/341,517, filed May 25, 2016; and 62/373,720, filed August 11, 2016.

### FIELD OF THE DISCLOSURE

The present disclosure relates to compositions for use in a method of treating at least one symptom of an anxiety disorder in a human subject who is diagnosed with both the anxiety disorder and a depression-related mood disorder.

### BACKGROUND

Anxiety disorders are a category of mental disorders that share features of excessive fear and anxiety and related behavioral disturbances. Different anxiety disorders have distinct symptom profiles, and major types include: generalized anxiety disorder (GAD), anxiety attacks (panic disorder), obsessive-compulsive disorder (OCD), phobia, social anxiety disorder, and post-traumatic stress disorder (PTSD).

Collectively, anxiety disorders are among the most common mental disorders in the United States, with a 12-month prevalence in the U.S. adult population of about 18%, and about 4% of the U.S. adult population having an anxiety disorder classified as being severe. (http://www.nimh.nih.gov/health/statistics/prevalence/any-anxiety-disorder-among-adults.shtml). Anxiety disorders are also a common health problem globally - results of a recent survey indicate that clinical anxiety affects about 10 percent of people in North America, Western Europe and Australia/New Zealand and about 8 percent in the Middle East and 6 percent in Asia. (Baxter, A.J. et al., Global prevalence of anxiety disorders: a systematic review and meta-regression. Psychol. Med. 2013 43:897-910.

Patients with anxiety disorders typically experience a combination of physical, emotional, cognitive and behavioral symptoms. A number of different medications are currently used to treat one or more symptoms of anxiety disorders. Since many people diagnosed with an anxiety disorder also suffer from depression, antidepressants such as selective serotonin reuptake inhibitors (SSRIs) and serotonin-norepinephrine reuptake inhibitors (SNRIs) are frequently used. Other medicines used to treat anxiety disorders include: anti-anxiety medications, such as benzodiazepines, which can help reduce the symptoms of anxiety panic attacks, or extreme fear and worry; and beta-blockers, such as propranolol and atenolol, which can be useful to control physical symptoms of anxiety such as rapid heartbeat, shaking, trembling and blushing in anxious situations. However, none of the currently used medications have optimal efficacy and side effect profiles.

There remains a need to identify additional medicaments and methods that offer the potential for improved efficacy and/or lower side effects in the treating the symptoms of anxiety disorders.

### SUMMARY

The present disclosure is based on clinical data showing that a hydrochloride salt of the compound of formula I (Compound I), 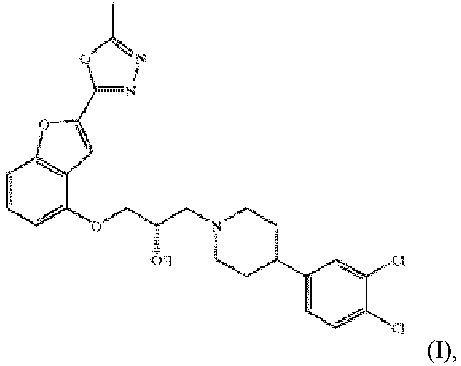 reduced anxiety symptoms compared to placebo in a cohort of adult subjects with major depressive disorder (MDD) as measured by change from Baseline in the Hamilton Anxiety Rating Scale (HAM-A) over 6 weeks of treatment. The HAM-A Scale is described in Hamilton M: The assessment of anxiety states by rating. British Journal of Medical Psychology. 32:50-55, 1959.

This hydrochloride salt of Compound I, having the chemical designation (2S)-1-[4-(3,4-dichlorophenyl)piperidin-1-yl]-3-[2-(5-methyl-1,3,4-oxadiazol-2-yl)benzo[*b*]furan-4-yloxy]propan-2-ol monohydrochloride, is named MIN-117 (formerly known as SON-117 and Wf-516) and is under clinical development by Minerva Neurosciences (Waltham, MA) for the treatment of major depressive disorder (MDD). The MIN-117 drug substance is an enantiomer with the (2S) configuration, a molecular formula of C₂₅H₂₅Cₗ₂N₃O₄·HCl and a molecular weight of 538.85. This investigational agent has been described as behaving as an antagonist on 5-HT_{1A} and 5-HT_{2A} receptors and both serotonin and dopamine reuptake, as well as having affinity for the α_{1A}- and α_{1B}-adrenergic receptors (Minerva Neurosciences, Inc., Form 10K, 14 March 2016).

The compound of formula II (Compound II) is a main metabolite of MIN-117 (M1): 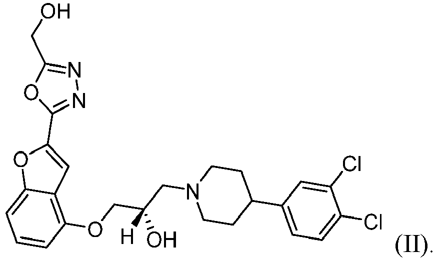 The chemical name for Compound II is (2*S*)-1-[4-(3,4-dichlorophenyl)piperidin-1-yl]-3-[2-(5-hydroxymethyl-1,3,4-oxadiazol-2-yl)benzo[*b*]furan-4-yloxy]propan-2-ol. The compounds of formula I and II have similar receptor and affinity binding profiles, and it is expected that the compounds of formula I and II will have similar exposures when administered to human subjects suffering from an anxiety disorder. Thus, it is expected that a compound of formula II, e.g., an isolated Compound II or a pharmaceutically acceptable salt, hydrate or solvate thereof, will produce reductions in anxiety symptoms in such subjects at similar doses as found useful for MIN-117. Unless otherwise specified, in the compositions, methods, or uses disclosed herein, Compound II or a pharmaceutically acceptable salt, hydrate, or solvate thereof, is in an isolated form, e.g., a purified form.

In one aspect, the disclosure provides compound of formula I (Compound I), 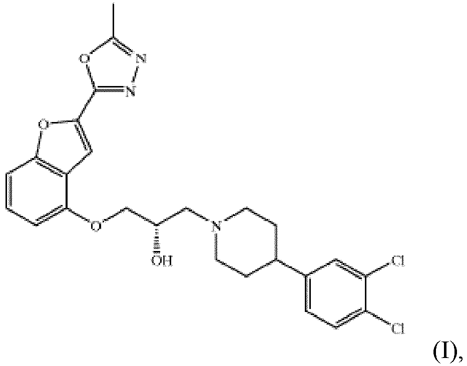 or a compound of formula II (Compound II) 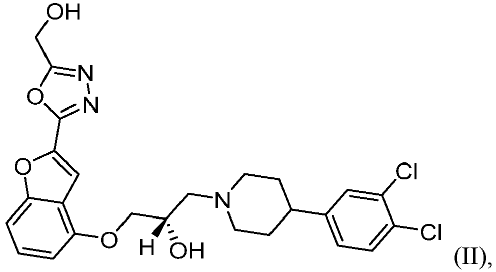 or a pharmaceutically acceptable salt, hydrate, or solvate of Compound I or II, for use in a method of treating at least one symptom of an anxiety disorder in a human subject who is diagnosed with both the anxiety disorder and a depression-related mood disorder, wherein the method comprises orally administering to the subject a total daily dose of Compound I or Compound II selected from the group consisting of:
(i) about 0.1 mg to less than 3.0 mg;
(ii) about 0.2 mg to about 2.9 mg;
(iii) about 0.3 mg to about 2.8 mg;
(iv) about 0.4 mg to about 2.7 mg;
(v) about 0.5 mg to about 2.6 mg;
(vi) about 0.6 mg to about 2.5 mg,
(vii) about 0.4 mg to about 0.6 mg; and
(viii) about 0.5 mg;
wherein the at least one symptom is a cognitive symptom of anxiety selected from the group consisting of: concentration problems, memory difficulties, and depressive symptoms.

In another aspect, the disclosure provides a composition comprising a compound of formula I (Compound I) 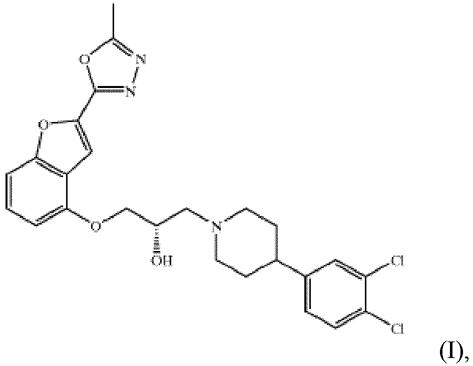 or a compound of formula II (Compound II) 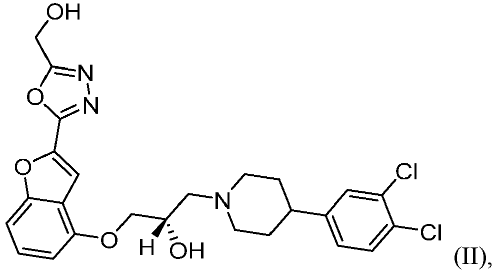 or a pharmaceutically acceptable salt, hydrate, or solvate of Compound I or Compound II, for use in a method for treating at least one symptom of an anxiety disorder in a human subject who is diagnosed with both the anxiety disorder and a depression-related mood disorder, wherein the method comprises orally administering to the subject a therapeutically effective amount of the composition, wherein the therapeutically effective amount is a total daily dose of Compound I or Compound II selected from the group consisting of:
(i) about 0.1 mg to less than 3.0 mg;
(ii) about 0.2 mg to about 2.9 mg;
(iii) about 0.3 mg to about 2.8 mg;
(iv) about 0.4 mg to about 2.7 mg;
(v) about 0.5 mg to about 2.6 mg;
(vi) about 0.6 mg to about 2.5 mg,
(vii) about 0.4 mg to about 0.6 mg; and
(viii) about 0.5 mg;
wherein the at least one symptom is a cognitive symptom of anxiety selected from the group consisting of: concentration problems, memory difficulties, and depressive symptoms.

In another aspect, the disclosure provides a monohydrochloride salt of a compound of formula I (Compound I), 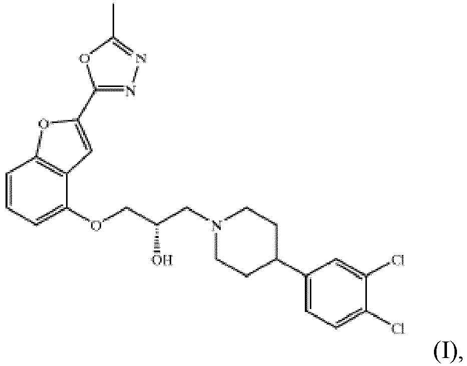 for use in a method of reducing symptoms of anxiety in an adult human subject, wherein the subject has been diagnosed with Major Depressive Disorder.

Further embodiments of the invention are set out in the appended dependent claims.

### BRIEF DESCRIPTION OF THE DRAWINGS

The foregoing summary, as well as the following detailed description, will be better understood when read in conjunction with the appended drawing.

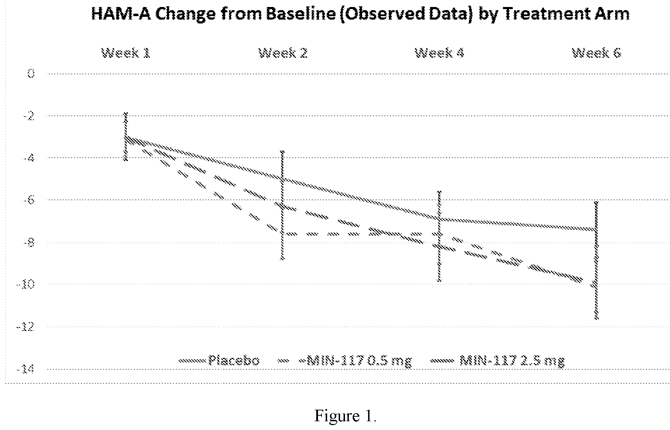

Figure 1 is a graph showing mean changes from baseline in total HAM-A scores (y-axis) in a population of MDD patients treated over six weeks with once daily administration of placebo (solid), 2.5 mg MIN-117 (long dashes) or 0.5 mg MIN-117 (short dashes).

### DETAILED DESCRIPTION

As described in the Examples set forth below, Compound I has been shown to have clinical activity in treating anxiety. Encompassed herein therefore are methods and compositions for treating various anxiety disorders. The references to methods of treatment in this treatment are to be interpreted as references to compounds and pharmaceutical compositions of the present disclosure for use in those methods.

U.S. Patent 6,720,320 discloses that a large number of phenoxypropylamine compounds and derivatives including Compound I and Compound II have selective affinity for and antagonistic activity against the 5-HT_{1A} receptor, as well as 5-HT reuptake inhibitory activity. Based on this *in vitro* activity, U.S. Patent 6,720,320 hypothesized that all of these compounds would be useful as a rapid onset antidepressant and for the treatment of a number of 5-HT mediated diseases of the central nervous system, including anxiety neurosis, OCD, panic disorder, social anxiety disorder (social phobia) and PTSD. However, no animal model data for any of the disclosed compounds or diseases is contained in U.S. Patent 6,720,320. Notably, U.S. Patent 6,720,320 states that the general daily dose of the described phenoxypropylamine compounds in the case of oral administration is a weight based dose range of 0.5-10 mg/kg, preferably 1-5 mg/kg.

A published patent application (US2014-0206722) discloses the use of Compound I for treating or diminishing symptoms of depression, and teach that effective treatment of selected disorders can occur with oral dosing that approaches 0.001 mg/kg. However, the use of Compound I for treating anxiety or any anxiety disorder is not mentioned in US2014-0206722.

A published patent application (US2002-111358) discloses the use of phenoxypropylamine compounds which show selective affinity for and antagonistic activity against 5-HT1A receptor, and 5-HT reuptake inhibitory activity, to express an anti-depressive effect.

Papp, M et al. in European Neuropsychopharmacology, Elsevier Science Publishers BV, Amsterdam NL, vol. 16, 1 January 2006, pages S305-S306, discloses the use of Compound I as an antagonist of 5-HT1A receptors and as an inhibitor of 5-HT reuptake in a chronic mild stress model of depression in rats.

Greibel, G et al. in Neuropharmacology, Pergamon Press, Oxford, GB, vol. 39, no. 10, 1 September 2000, pages 1848-1857, discloses the effects of compounds varying in selectivity as 5-HT1A receptor antagonists in three rat models of anxiety.

It has now been unexpectedly observed that total oral daily doses of 0.5 mg and 2.5 mg of Compound I are capable of reducing the severity of one or more anxiety symptoms in patients diagnosed with MDD as measured on the HAMA-A scale, with the 0.5 mg dose demonstrating a more rapid reduction in the total HAMA-A score than the 2.5 mg dose. For purposes of reference, a 2.5 mg dose for a 70 kg subject equates to a weight-based dose of 0.036 mg/kg, which is more than 10 times lower than the lowest oral daily dose mentioned in U.S. Patent 6,720,320.

Thus, it is an object of the present disclosure to provide a method of treating at least one symptom of an anxiety disorder in a human subject diagnosed with the anxiety disorder comprising administering to the subject a therapeutically effective amount of a composition comprising Compound I or Compound II or a pharmaceutically acceptable salt, hydrate, or solvate of Compound I or Compound II.

It is also an object of the present disclosure to provide a composition comprising Compound I of Compound II, or a pharmaceutically acceptable salt, hydrate, or solvate of Compound I or Compound II, for use in a method for treating at least one symptom of an anxiety disorder in a human subject diagnosed with the anxiety disorder, wherein the method comprises administering to the subject a therapeutically effective amount of the composition.

It is a further object of the present disclosure to employ the compositions and methods of the disclosure to treat at least one symptom of an anxiety disorder in a human subject diagnosed with an anxiety disorder and with a co-morbid mental disorder, such as depression.

In embodiments, unless otherwise specified, Compound II, or a pharmaceutically acceptable salt, hydrate, or solvate thereof is in an isolated form.

As used herein, the term "isolated" or the expression "in an isolated form" means substantially separated from other components with which the compound may be found as it occurs in nature. A compound can be isolated without necessarily being purified. In one embodiment, the isolated compound of formula I or II herein (or Compound I or Compound II) is a synthesized compound. In another embodiment, Compound II is a metabolite and is isolated from other components with which the compound may be found as it occurs in a natural environment, e.g., cells of a mammal, e.g., a human or appropriate non-human mammal, such as primate, mouse, rat, dog, cat, cow, horse, goat, and camel. In embodiments, the compounds or salts, hydrates, or solvates thereof disclosed herein each have a purity of not less than 40%, not less than 50%, not less than 55%, not less than 60%, not less than 65%, not less than 70%, not less than 75%, not less than 80%, not less than 85%, not less than 90%, not less than 92%, not less than 95%, not less than 97%, or not less than 99%.

As used herein, unless otherwise noted, the terms "treat", "treating", "treatment" and the like, shall include the management and care of a subject for the purpose of combating an anxiety disorder and include administration of Compound I or Compound II in an amount and for a treatment period that are sufficient to prevent the onset of one or more symptoms of an anxiety disorder, reduce the frequency, intensity or severity of one or more symptoms of an anxiety disorder, delay or avoid the development of additional symptoms, or any combination of these treatment objectives. In an embodiment, the effect of treatment with Compound I or Compound II is assessed by comparing the severity of the subject's anxiety at baseline (e.g., prior to treatment with Compound I or II) and after at least one treatment period. In an embodiment, the treatment period is at least one week, at least two weeks, at least four weeks, at least six weeks, at least eight weeks, or at least twelve weeks.

In an embodiment, the effect of the treatment is measured using the Hamilton Anxiety Scale (HAM-A). The HAM-A is composed of 14 items, each defined by a series of symptoms as shown in Table 1 below. The subject is asked to rate the gravity of each item using a 5-level scale - from 0 to 4, where 0 being not present and 4 being severe. The results are then collated and tabulated to determine the severity of anxiety: a HAM-A score of <17 is mild anxiety, HAM-A score of 18-24 is mild to moderate anxiety, and HAM-A score of 25-30 is moderate to severe anxiety.

**Table 1: Hamilton Anxiety Scale (HAM-A)**

| **1. Anxious mood** | |
|---|---|
| This item covers the emotional condition of uncertainty about the future, ranging from worry, insecurity, irritability and apprehension to overpowering dread. | |
| **0** - The patient is neither more or less insecure or irritable than usual. | □ |
| **1** - Doubtful whether the patient is more insecure or irritable than usual. | □ |
| **2** - The patient expresses more clearly to be in a state of anxiety, apprehension or irritability, which he may find difficult to control. However, the worrying still is about minor matters and thus without influence on the patient's daily life. | □ |
| **3** - At times the anxiety or insecurity is more difficult to control because the worrying is about major injuries or harms which might occur in the future. Has occasionally interfered with the patient's daily life. | □ |
| **4** - The feeling of dread is present so often that it markedly interferes with the patient's daily life. | □ |
| | |

| **2. Tension** | |
|---|---|
| This item includes inability to relax, nervousness, bodily tensions, trembling and restless fatigue. | |
| **0** - The patient is neither more nor less tense than usual. | □ |
| **1** - The patient seems somewhat more nervous and tense than usual. | □ |
| **2** - Patient expresses clearly unable to relax and full of inner unrest, which he finds difficult to control, but it is still without influence on the patient's daily life. | □ |
| **3** - The inner unrest and nervousness is so intense or frequent that it occasionally interferes with the patient's daily work. | □ |
| **4** - Tensions and unrest interfere with the patient's life and work at all times. | □ |

| **3. Fears** | |
|---|---|
| This item includes fear of being in a crowd, of animals, of being in public places, of being alone, of traffic, of strangers, of dark etc. It is important to note whether there has been more phobic anxiety during the present episode than usual. | |
| **0 -** Not present. | □ |
| **1** - Doubtful whether present. | □ |
| **2** - The patient experiences phobic anxiety but is able to fight it. | □ |
| **3** - It is difficult to fight or overcome the phobic anxiety, which thus to some extent interferes with the patient's daily life and work. | □ |
| **4** - The phobic anxiety clearly interferes with the patient's daily life and work. | □ |
| | |

| **4. Insomnia** | |
|---|---|
| This item covers the patient's subjective experience of sleep duration and sleep depth during the three preceding nights. Note: Administration of hypnotics or sedatives is disregarded. | |
| **0** - Usual sleep duration and sleep depth. | □ |
| **1** - Sleep duration is doubtfully or slightly reduced (e.g. due to difficulties falling asleep), but no change in sleep depth. | □ |
| **2** - Sleep depth is also reduced, sleep being more superficial. Sleep as a whole is somewhat disturbed. | □ |
| **3** - Sleep duration and sleep depth is markedly changed. Sleep periods total only a few hours per 24 hours. | □ |
| **4** - Sleep depth is so shallow that the patient speaks of short periods of slumber or dozing, but no real sleep. | □ |

| **5. Difficulties in concentration and memory** | |
|---|---|
| This item covers difficulties in concentration, making decision about everyday matters, and memory. | |
| **0 -** The patient has neither more nor less difficulty in concentration and/or memory that usual. | □ |
| **1** - Doubtful whether the patient has difficulty in concentration and/or memory. | □ |
| **2** - Even with a major effort it is difficult for the patient to concentrate on his daily routine work. | □ |
| **3** - The patient has pronounced difficulties with concentration, memory, or decision making, e.g. in reading a newspaper article or watching a television program to the end. | □ |
| **4** - During the interview the patient shows difficulty in concentration, memory or decision making. | □ |
| | |

| **6. Depressed mood** | |
|---|---|
| This item covers both the verbal and the non-verbal communication of sadness, depression, despondency, helplessness and hopelessness. | |
| **0 -** Not present. | □ |
| **1** - Doubtful whether the patient is more despondent or sad than usual, or is only vaguely so. | □ |
| **2** - The patient is more clearly concerned with unpleasant experiences, although he still lacks helplessness or hopelessness. | □ |
| **3** - The patient shows clear non-verbal signs of depression and/or hopelessness. | □ |
| **4** - The patient remarks on despondency and helplessness or the non-verbal signs dominate the interview and the patient cannot be distracted. | □ |

| **7. General somatic symptoms: Muscular** | |
|---|---|
| Weakness, stiffness, soreness or real pain, more or less diffusely localized in the muscles, such as jaw ache or neck ache. | |
| **0** - The patient is neither more nor less sore or stiff in the muscles than usual. | □ |
| **1** - The patient seems somewhat more stiff or sore in the muscles than usual. | □ |
| **2** - The symptoms have the character of pain. | □ |
| **3** - Muscle pain interferes to some extent with the patient's daily work and life. | □ |
| **4** - Muscle pain is present most of the time and clearly interferes with the patient's daily work and life. | □ |
| | |

| **8. General somatic symptoms: Sensory** | |
|---|---|
| This item includes increased fatigability and weakness or real functional disturbances of the senses, including tinnitus, blurring of vision, hot and cold flashes and prickling sensations. | |
| **0 -** Not present. | □ |
| **1** - Doubtful whether the patient's indications of symptoms are more pronounced than usual. | □ |
| **2** - The sensations of pressure reach the character of buzzing in the ears, visual disturbances and prickling or itching sensations in the skin. | □ |
| **3** - The generalized sensory symptoms interfere to some extent with the patient's daily life and work. | □ |
| **4** - The generalized sensory symptoms are present most of the time and clearly interfere with the patient's daily life and work. | □ |

| **9. Cardiovascular symptoms** | |
|---|---|
| This item includes tachycardia, palpitations, oppression, chest pain, throbbing in the blood vessels, and feelings of faintness. | |
| **0 -** Not present. | □ |
| **1** - Doubtful whether present. | □ |
| **2** - Cardiovascular symptoms are present, but the patient can still control them. | □ |
| **3** - The patient has occasional difficulty controlling the cardiovascular symptoms, which thus to some extent interfere with his daily life and work. | □ |
| **4** - Cardiovascular symptoms are present most of the time and clearly interfere with the patient's daily life and work. | □ |
| | |

| **10. Respiratory symptoms** | |
|---|---|
| Feelings of constriction or contraction in throat or chest, dyspnoea or choking sensations and sighing respiration. | |
| **0 -** Not present. | □ |
| **1** - Doubtful whether present. | □ |
| **2** - Respiratory symptoms are present, but the patient can still control them. | □ |
| **3** - The patient has occasional difficulty controlling the respiratory symptoms, which thus to some extent interfere with his daily life and work. | □ |
| **4** - Respiratory symptoms are present most of the time and clearly interfere with the patient's daily life and work. | □ |

| **11. Gastro-intestinal symptoms** | |
|---|---|
| This item covers difficulties in swallowing, "sinking" sensation in stomach, dyspepsia (heartburn or burning sensation in the stomach, abdominal pains related to meals, fullness, nausea and vomiting), abdominal rumbling and diarrhea. | |
| **0 -** Not present. | □ |
| **1** - Doubtful whether present (or doubtful whether different from usual). | □ |
| **2** - One or more gastro-intestinal symptoms are present, but the patient can still control them. | □ |
| **3** - The patient has occasional difficulty controlling the gastro-intestinal symptoms, which to some extent interfere with his daily life and work. | □ |
| **4** - The gastro-intestinal symptoms are present most of the time and interfere clearly with the patient's daily life and work. | □ |
| | |

| **12. Genito-urinary symptoms** | |
|---|---|
| This item includes non-organic or psychic symptoms such as frequent or more pressing passing of urine, menstrual irregularities, anorgasmia, dyspareunia, premature ejaculation, loss of erection. | |
| **0 -** Not present. | □ |
| **1** - Doubtful whether present (or doubtful whether different from usual). | □ |
| **2** - One or more genito-urinary symptoms are present, but do not interfere with the patient's daily life and work. | □ |
| **3** - Occasionally, one or more genito-urinary symptoms are present to such a degree that they interfere to some extent with the patient's daily life and work. | □ |
| **4** - The genito-urinary symptoms are present most of the time and interfere clearly with the patient's daily life and work. | □ |

| **13. Other autonomic symptoms** | |
|---|---|
| This item includes dryness of the mouth, blushing or pallor, sweating and dizziness. | |
| **0** - Not present. | □ |
| **1** - Doubtful whether present. | □ |
| **2** - One or more autonomic symptoms are present, but they do not interfere with the patient's daily life and work. | □ |
| **3** - Occasionally, one or more autonomic symptoms are present to such a degree that they interfere to some extent with the patient's daily life and work. | □ |
| **4** - Autonomic symptoms are present most of the time and clearly interfere with the patient's daily life and work. | □ |
| | |

| **14. Behavior during interview** | |
|---|---|
| The patient may appear tense, nervous, agitated, restless, tremulous, pale, hyperventilating or sweating during the interview. Based on such observations a global estimate is made. | |
| **0 -** The patient does not appear anxious. | □ |
| **1** - It is doubtful whether the patient is anxious. | □ |
| **2** - The patient is moderately anxious. | □ |
| **3** - The patient is markedly anxious. | □ |
| **4** - Patient is overwhelmed by anxiety, for example with shaking and trembling all over. | □ |
| **Total score** ______ | |

As used herein, the terms "subject" and "patient" may be used interchangeably, and refer to a human of any age. In an embodiment, the subject is six or more years of age. In some embodiments, the subject is at least 18, 19, 20 or 21 years of age. The subject is diagnosed with an anxiety disorder. In some examples, the subject is not diagnosed with another co-morbid mental disorder. In embodiments of the invention, the subject is diagnosed with a co-morbid mental disorder. In embodiments of the invention, the co-morbid mental disorder is a depression-related mood disorder.

In some embodiments, a composition or method of the disclosure is used to treat a subject diagnosed with an anxiety disorder who is treatment-naive to an anxiolytic drug. As used herein, an anxiolytic drug is any drug that does not contain Compound I or Compound II and has been approved by a regulatory agency for the treatment of anxiety or an anxiety disorder.

In other embodiments, a composition or method of the disclosure is used to treat a subject diagnosed with an anxiety disorder who was previously treated with an anxiolytic drug but discontinued such treatment, e.g., because the drug did not provide adequate control of the subject's anxiety symptoms and / or because the subject could not tolerate the side effects of the drug.

For purposes of the disclosure encompassed herein, the term "anxiety disorder" is to be understood as encompassing those conditions defined as anxiety disorders in the Diagnostic and Statistical Manual of Mental Disorders, Fourth or Fifth Editions, published in 2000 ("DSM-IV-TR") and 2013 (DSM-5), respectively.

In an example, the at least one symptom of an anxiety disorder that is treated using the compositions and methods of the present disclosure is selected from the group consisting of panic attacks, physical symptoms of anxiety, emotional symptoms of anxiety, behavioral symptoms of anxiety and cognitive symptoms of anxiety. As will be evident to the skilled artisan, a subject with one or more of these symptoms of anxiety would not typically be considered to have an anxiety disorder unless the symptom(s) reach a level of intensity, duration and frequency such that the symptoms cause significant distress to the subject and / or interfere with the subject's functioning.

In an example, the anxiety disorder symptom is a panic attack, which is a sudden, extreme feeling of fear and/or discomfort lasting for a distinct period of time. Typically, the subject will be diagnosed with a panic attack if the subject has at least 4 of the following symptoms: palpitations and/or pounding heart; sweating; trembling or shaking; shortness of breath or a sense of being smothered; feelings of choking; chest pain or discomfort, nausea; feeling dizzy, unsteady, lightheaded, or faint; hot flashes or chills; numbness or tingling sensation; derealization (feelings of unreality), depersonalization (feeling detached from oneself); fear of losing control; and fear of dying. In some examples, the subject suffers from unexpected (uncued) panic attacks, which do not have an identifiable source that triggers a panic attack. While in other examples, the subject suffers from expected (cued) panic attacks, i.e., attacks with an obvious cue or trigger.

In an example, the anxiety disorder symptom is a physical symptom selected from the group consisting of: restlessness, shortness of breath, choking feeling, sweaty palms, racing heart, chest pain or discomfort, "butterflies" in the stomach, muscle tension, trembling, twitches, nausea, diarrhea, dizziness, feeling faint, hot flashes, chills, numbness, tingling sensations, an exaggerated startle response, headaches, sleep disturbance, and fatigue.

In an example, the anxiety disorder symptom is an emotional symptom selected from the group consisting of: feelings of apprehension or dread, distress, dread, nervousness, feeling overwhelmed, panic, uneasiness, worry, fear or terror, jumpiness or edginess, irritability, and feeling like the mind has gone blank.

In an example, the anxiety disorder symptom is a behavioral symptom of anxiety selected from the group consisting of: anxious avoidance, which refers to avoiding anxiety-producing situations (e.g., social gatherings) or places (e.g., using the stairs instead of an elevator); escaping from an anxiety-producing situation or place; sensitization, which is a coping strategy in which the subject seeks to learn about, rehearse, and/or anticipate fearful events; safety behaviors, in which the subject becomes overly attached to a safety object or person (e.g., refusing to go out, away from home, to school, or to work in order to avoid separation); engaging in unhealthy, risky or self-destructive behaviors (e.g., excessive drinking or drug use to deal with the anxiety); and feeling compelled to limit the amount and scope of one's daily activities to reduce the overall level of anxiety (e.g., remaining in the subject's home).

In embodiments of the invention, the anxiety disorder symptom is a cognitive symptom of anxiety selected from the group consisting of: concentration problems (i.e., difficulty with staying on task); memory difficulties; and depressive symptoms (e.g., hopelessness, lethargy, poor appetite).

In an embodiment, a composition or method of the disclosure is used to treat a human subject diagnosed with generalized anxiety disorder (GAD). In an example, and the symptom of GAD treated is selected from the group consisting of chronic worrying, a general feeling of dread or unease, feeling like the anxiety is uncontrollable, inability to tolerate uncertainty, nervousness, tension, insomnia, nausea, diarrhea, restlessness and fatigue.

In an embodiment, a composition or method of the disclosure is used to treat a human subject diagnosed with panic disorder, which is characterized by repeated, unexpected panic attacks, that are followed for at least a month by constant concerns about having more attacks and worrying about the consequences of the attacks.

In an embodiment, a composition or method of the disclosure is used to treat a human subject diagnosed with a phobia. In an embodiment, the phobia is agoraphobia, a specific phobia or social phobia (e.g., Social Anxiety Disorder). Typically, a subject must have symptoms that persist for at least 6 months to be diagnosed as having a phobia.

Exemplary symptoms of agoraphobia include intense fear, anxiety and avoidance of non-specific situations where escape of difficult or help might be unavailable if a panic attack occurs. Typically, a subject is diagnosed with agoraphobia if these symptoms occur in at least two of the following situations: using public transportation, being in open spaces, being in enclosed spaces, standing in line or being in a crowd, being outside of the home alone.

Exemplary symptoms of a specific phobia include intense fear, anxiety and avoidance of a specific object or situation that is out of proportion to any real danger posed by the object or situation, such as flying, heights, injections, animals.

Exemplary symptoms of social phobia include intense fear, anxiety and avoidance of social situations where there is the potential of being scrutinized or negatively judged by others.

In an embodiment, a composition or method of the disclosure is used to treat a human subject diagnosed with selective mutism, which is characterized by persistent refusal, typically for at least one month, to speak in specific situations where speaking is expected.

The compositions and methods of the disclosure may also be used to treat one or more symptoms of anxiety in a patient diagnosed with both an anxiety disorder and a depression-related mood disorder. In an embodiment, the depression related mood-related disorder is selected from the group consisting of major depressive disorder, persistent depressive disorder, depression associated with a bipolar disorder, dysthymic disorder, cyclothymic disorder, and substance-induced mood disorder.

The methods of the disclosure employ administering to the subject a therapeutically effective amount of Compound I or Compound II, or a pharmaceutically acceptable salt, hydrate, or solvate of Compound I or II. As used herein, the term "therapeutically effective amount" means an amount that is effective to reduce the presence and/or severity of at least one anxiety symptom by at least 10%, at least 20%, at least 40%, or at least 80% compared to baseline. Anxiety symptoms in the subject may be measured using any measurement tool generally accepted in the art, including but not limited to the HAM-A scale, the State-Trait Anxiety Inventory (STAI, available from Mind Garden, 855 Oak Grove Avenue, Suite 215, Menlo Park, CA 94025, http://www.mindgarden.com/index.htm), the Beck Anxiety Inventory (BAI, available at www.pearsonassessments.com), the Hospital Anxiety and Depression Scale-Anxiety (HADS-A, available from: Nfer Nelson, The Chiswick Centre, 414 Chiswick High Road, London W4 5TF United Kingdom, www.nfer-nelson.co.uk).

In some embodiments of the compositions and methods of the disclosure, the dose of Compound I or Compound II that is administered to a subject diagnosed with an anxiety disorder is effective to reduce the total HAMA-A score from baseline by at least 25%, at least 50%, or at least 75% after at least 1 week, at least 2 weeks, at least 3 weeks, at least 4 weeks, at least 5 weeks or at least 6 weeks of treatment.

In yet another aspect of the disclosure, a composition of the disclosure is formulated and administered to the subject in a manner that provides a dose of Compound I or Compound II that is substantially equivalent to oral administration of any of the total daily doses specifically described herein. The skilled artisan can readily select formulations and administration routes that would provide such functional equivalence.

The disclosure also provides use of Compound I or Compound II or a pharmaceutically acceptable salt, hydrate, solvate or polymorph thereof, in the manufacture of a medicament for treating at least one symptom of an anxiety disorder in a human subject who is diagnosed with the anxiety disorder. For example, the medicament is suitable for oral administration. For example, the medicament has a therapeutically effective amount of Compound I, which corresponds to a total daily dose of Compound I of between about 0.5 mg to about 2.5 mg.

It will be understood by the skilled artisan that the treating physician may select a dose and dosing regimen within the above guidelines that he or she believes is appropriate based on the health and condition of the subject to be treated, as well as the desired outcome of the treatment. For example, the treating physician may choose to start therapy with a lower than therapeutically effective dose of Compound I or Compound II and titrate up to a target therapeutically effective dose. For example, the total daily dose of Compound I or Compound II may be administered in a single dose or in multiple doses.

As used herein, quantitative expressions recited as a range of from about value X to about value Y include any value that is 10% higher or lower than each of X and Y, and also includes any numerical value that falls between X and Y. Thus, for example, a dose of about 0.5 mg includes a dose of between 0.45 to 0.55 mg.

All references to Compound I and Compound II herein include all pharmaceutically acceptable salts and all solvates and alternative physical forms thereof unless otherwise states. All doses recited herein for Compound I are based on the weight of the free base of Compound I, rather than the pharmaceutically acceptable salt, hydrate of solvate thereof or any excipients in the composition, unless otherwise stated. Similarly, all doses recited herein for Compound II are based on the weight of the free base of Compound II, rather than the pharmaceutically acceptable salt, hydrate of solvate thereof or any excipients in the composition, unless otherwise stated. Further, all doses of Compound I or II recited herein are flat doses (e.g., not dependent on weight of the patient) unless otherwise stated.

For therapeutic administration according to the present disclosure, Compound I or Compound II may be employed in the form of its free base, but is preferably used in the form of a pharmaceutically acceptable salt. In an embodiment, the form of Compound I used in the compositions and methods of the disclosure is (2S)-1-[4-(3,4-dichlorophenyl)piperidin-1-yl]-3-[2-(5-methyl-1,3,4-oxadiazol-2-yl)benzo[*b*]furan-4-yloxy]propan-2-ol monohydrochloride. In an embodiment, the form of Compound II used in the compositions and methods of the disclosure is (2*S*)-1-[4-(3,4-dichlorophenyl)piperidin-1-yl]-3-[2-(5-hydroxymethyl-1,3,4-oxadiazol-2-yl)benzo[*b*]furan-4-yloxy]propan-2-ol monohydrochloride.

Compound (I) and Compound (II) may be synthesized using standard synthetic methods and procedures for the preparation of organic molecules and functional group transformations and manipulations, including the use of protective groups, as can be obtained from the relevant scientific literature or from standard reference textbooks in the field. Although not limited to any one or several sources, recognized reference textbooks of organic synthesis include: Smith, M. B.; March, J. March's Advanced Organic Chemistry: Reactions, Mechanisms, and Structure, 5th ed.; John Wiley & Sons: New York, 2001; and Greene, T.W.; Wuts, P.G. M. Protective Groups in Organic Synthesis, 3rd; John Wiley & Sons: New York, 1999. A method for preparing Compound (I) is described in U.S. Patent No. 6,720,320.

In an embodiment, alternative salts of Compound I or Compound II with pharmaceutically acceptable acids may also be utilized in therapeutic administration, for example salts derived from the functional free base and acids including, but not limited to, palmitic acid, hydrobromic acid, phosphoric acid, acetic acid, fumaric acid, maleic acid, salicylic acid, citric acid, oxalic acid, lactic acid, malic acid, methanesulfonic acid and p-toluene sulfonic acid.

All solvates and all alternative physical forms of Compound I, Compound II or their pharmaceutically acceptable derivatives as described herein, including but not limited to alternative crystalline forms, amorphous forms and polymorphs, are also within the scope of this disclosure, and all references to a compound of formula I or II herein (or Compound I or Compound II) include all pharmaceutically acceptable salts, and all solvates and alternative physical forms thereof.

For therapeutic administration, Compound I or Compound II, or a pharmaceutically acceptable salt of Compound I or II, for example, the HCl salt, may be administered in pure form, but will preferably be formulated into any suitable pharmaceutically acceptable and effective composition which provides effective levels of the active ingredient in the body.

The term "pharmaceutically acceptable", as used herein with respect to a compound or composition, refers to a form of the compound or composition that can increase or enhance the solubility or availability of the compound in a subject, in order to promote or enhance the bioavailability of the compound or composition. In an example, the disclosure herein also encompasses pharmaceutically acceptable, hydrates, solvates, stereoisomers, or amorphous solids of the compounds and compositions embodied herein. For example, the term "pharmaceutically acceptable salt" is to describe a salt form of one or more of the compositions herein which are presented to increase the solubility of the compound, for example, in the gastric juices of the patient's gastrointestinal tract in order to promote dissolution and the bioavailability of the compounds and/or compositions. In an embodiment, pharmaceutically acceptable salts include those derived from pharmaceutically acceptable inorganic or organic bases and acids. Suitable salts include those derived from alkali metals such as potassium and sodium, alkaline earth metals such as calcium, magnesium and ammonium salts, among numerous other acids well known in the pharmaceutical art. Sodium and potassium salts are particularly preferred as neutralization salts of carboxylic acids and free acid phosphate containing compositions encompassed by the present disclosure. The term "salt" shall mean any salt consistent with the use of the compounds encompassed by the present disclosure. In the case where the compounds are used in pharmaceutical indications, including the treatment of depression, the term "salt" shall mean a pharmaceutically acceptable salt, consistent with the use of the compounds as pharmaceutical agents.

The term "pharmaceutically acceptable derivative" or "derivative", as used herein, describes any pharmaceutically acceptable prodrug form (such as an ester or ether or other prodrug group) which, upon administration to a patient, provides directly or indirectly the present compound or an active metabolite of the present compound.

As set forth above, the compositions include pharmaceutically acceptable salts of the compounds in the composition. In other embodiments, the acids which are used to prepare the pharmaceutically acceptable acid addition salts of the aforementioned compounds are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3 naphthoate)] salts, among others.

In an embodiment, compositions comprise base addition salts of the present compounds. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine (meglumine), and the lower alkanolammonium and other base salts of pharmaceutically acceptable organic amines, among others.

As used herein, the term pharmaceutically acceptable salts or complexes refers to salts or complexes (e.g., solvates, polymorphs) that retain the desired biological activity of the parent compound and exhibit minimal, if any, undesired toxicological effects. Non-limiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acids, naphthalenedisulfonic acids, and polygalacturonic acid; (b) base addition salts formed with polyvalent metal cations such as zinc, calcium, bismuth, barium, magnesium, aluminum, copper, cobalt, nickel, cadmium, sodium, potassium, and the like, or with an organic cation formed from N,N-dibenzylethylene-diamine, ammonium, or ethylenediamine; or (c) combinations of (a) and (b); e.g., a zinc tannate salt or the like.

Modifications of a compound can affect the solubility, bioavailability and rate of metabolism of the active species, thus providing control over the delivery of the active species. Further, the modifications can affect the anxiolytic activity of the compound, in some cases increasing the activity over the parent compound. This can easily be assessed by preparing the derivative and testing its activity according to the methods encompassed herein, or other methods known to those skilled in the art.

In an embodiment, the compositions may be formulated in a conventional manner using one or more pharmaceutically acceptable carriers and may also be administered in controlled-release formulations. Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions encompassed herein may be administered orally. In other embodiments, compositions may be administered parenterally, by inhalation spray, topically, rectally, nasally, buccally, vaginally or via an implanted reservoir. The term "parenteral" as used herein includes subcutaneous, percutaneous, intravenous, intramuscular, intra-articular, intra-synovial, intrasternal, intrathecal, intrahepatic, intralesional and intracranial injection or infusion techniques. As will be understood by the skilled artisan, in view of the embodiments encompassed herein, the dosage of active ingredient or ingredients (e.g., a compound of formula I) may be adjusted upward or downward based on the selected route of administration. Furthermore, it will be understood that optimizing the dosage of active ingredient for any selected dosage form may be desired and can be achieved by using the methods described herein or known in the art to evaluate the effectiveness of anxiolytic compounds.

The pharmaceutical compositions embodied herein may be orally administered in any orally acceptable dosage form including, but not limited to, capsules, tablets, aqueous suspensions or solutions. In the case of tablets for oral use, carriers which are commonly used include lactose and corn starch. In an embodiment, lubricating agents, such as magnesium stearate, are also added. For oral administration in a capsule form, useful diluents include lactose and/or dried corn starch, as two non-limiting examples. When aqueous suspensions are required for oral use, the active ingredient is combined with emulsifying and suspending agents. If desired, certain sweetening, flavoring or coloring agents may also be added.

The pharmaceutical compositions encompassed by the present disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In an embodiment, the therapeutically effective amount of Compound I is administered independently of any other medication that is indicated for the treatment of a mental disorder.

In another embodiment, the therapeutically effective amount of Compound I is administered in conjunction with one or more other medications to treat the anxiety disorder or to treat a co-morbid medical condition, including a depression-related mood disorder. Such other medications may be administered or co-administered in forms and dosages as known in the art, or in the alternative, as has been described above for administration of Compound I. The other medication(s) may be administered before, after or simultaneously with Compound I during a desired treatment period.

In an embodiment, the therapeutically effective amount of Compound I may be administered to a patient diagnosed with a depression-related mood disorder and who is already undergoing treatment with an anti-depressant.

In an embodiment, the therapeutically effective amount of Compound II is administered independently of any other medication that is indicated for the treatment of a mental disorder.

In another embodiment, the therapeutically effective amount of Compound II is administered in conjunction with one or more other medications to treat the anxiety disorder or to treat a co-morbid medical condition, including a depression-related mood disorder. Such other medications may be administered or co-administered in forms and dosages as known in the art, or in the alternative, as has been described above for administration of Compound II. The other medication(s) may be administered before, after or simultaneously with Compound II during a desired treatment period.

In an embodiment, the therapeutically effective amount of Compound II may be administered to a patient diagnosed with a depression-related mood disorder and who is already undergoing treatment with an anti-depressant.

In an embodiment, a compound set forth herein can be co-administered with one or more atypical antipsychotics. Examples of atypical antipsychotics include, but are not limited to fluphenazine, risperidone, olanzapine, clozapine, quetiapine, ziprasidone, aripiprazole, sertindole, zotepine, and perospirone. Examples of antidepressants useful in combination therapy as encompassed herein include, but are not limited to, fluoxetine, citalopram, escitalopram, venlafaxine, duloxetine and bupropion.

### EXAMPLES

Example 1. MIN-117 reduces anxiety symptoms in patients diagnosed with MDD.

A phase 2a, multicenter, multinational, randomized, double-blind, placebo- and active-controlled parallel-group study was conducted to investigate the efficacy and safety of MIN-117 in adult subjects with MDD. One of the exploratory objectives of the study was to assess effects of MIN-117 compared to placebo on anxiety as measured by change from baseline in the Hamilton Anxiety Rating Scale (HAM-A) over 6 weeks of treatment.

The study consisted of a 4-week pre-treatment phase during which subjects were washed out from antidepressant medications or other psychotropics, followed by a 6-week treatment phase, and then an end of study (EOS) follow-up visit at approximately 2-weeks after the end of the treatment phase. MIN-117 capsules of 0.5 mg and 2.5 mg strength were used in the study. The capsules were size 4 made of white hard gelatin containing the drug substance, lactose, cornstarch, low substituted hydroxypropylcelullose and hydroxypropylcelullose, and purified water.

Eighty-four eligible subjects (met the DSM-5 diagnostic criteria for moderate to severe MDD, without psychotic features) were randomized in a 1:1:1:1 ratio, to receive once daily oral doses of placebo, MIN-117 at 0.5 mg, MIN-117 at 2.5 mg, or paroxetine at 20 mg as an active comparator for 6 weeks. Paroxetine is an antidepressant of the SSRI class and is used to treat MDD, OCD, PTSD, social anxiety disorder, panic disorder, GAD, premenstrual dysphoric disorder and menopausal hot flashes. The HAM-A scale was used to measure the severity of each subject's anxiety at screening, baseline, and weeks 1, 2, 4, 6 (or early withdrawal) and EOS.

The Inclusion and Exclusion Criteria for the study are set forth below.

### Inclusion Criteria

Subjects had to meet all of the following inclusion criteria to be eligible for participation in this study:
1. Subjects must be able to read and understand the consent forms, complete study-related procedures, and communicate with the study staff.
2. Subjects must have provided written consent to participate in the study and understand that they are free to withdraw from the study at any time.
3. Male or female subjects must be 18 to 65 years of age, inclusive, at Screening (Visit 1).
4. Subjects have a body mass index (BMI) of > 18 to ≤ 35 kg/m² [BMI = weight (kg)/height (m²)] at Screening (Visit 1)
5. Subjects must meet Diagnostic and Statistical Manual of Mental Disorders - Fifth Edition (DSM-5) criteria for diagnosis of moderate or severe depression without psychotic features (ICD-9 codes 296.32 & 296.33; ICD-10 codes F33.1 & F33.2, respectively) at Screening based on clinical assessment and on the MINI, v7.0.
6. Subjects have a history of at least one previous episode of depression prior to the current episode.
7. Current major depressive episode of at least 8 weeks in duration.
8. Subjects must have a score of ≥ 30 on the investigator-rated MADRS at Screening (Visit 1) and Baseline (Visit 2a).
9. Subjects must have a score of ≥ 4 on the investigator-rated CGI-S at Screening (Visit 1) and Baseline (Visit 2a).
10. Subjects must be in good general health prior to study participation with no clinically relevant abnormalities as assessed by the investigator and determined by medical history, physical examination, blood chemistry, hematology, urinalysis, and electrocardiogram (ECG).
11. Female subjects must be postmenopausal, have had a hysterectomy or tubal ligation or be otherwise incapable of pregnancy, or must agree to consistent use of 2 approved methods of contraception for the duration of the study (oral or parenteral hormonal contraceptive or intrauterine device or barrier plus spermicide) and until 90 days after the last dose of the study treatment.
12. Male subjects who are willing to use condom for the duration of the study and until 90 days after the last dose of the study, even if they have had vasectomy, and must inform their partners about taking an investigational medication. Female partners of subjects who have not had vasectomy must also agree to use an approved method of contraception until 90 days after the last dose of the study treatment.

### Exclusion Criteria

Subjects who met any of the following criteria were to be excluded from participating in this study:
1. A DSM-5 diagnosis of ***current*** (active) obsessive compulsive disorder (OCD), post-traumatic stress disorder (PTSD), anorexia nervosa, or bulimia nervosa.
2. Significant past or present neurological or neurosurgical disorder that could interfere with the study assessments (including but not limited to stroke, central nervous system [CNS]-related tumors, multiple sclerosis, Parkinson's disease, Alzheimer's disease, Huntington's disease, seizure disorder requiring current anticonvulsants, history of brain injury or trauma, or neurosyphilis).
3. History or current diagnosis of schizophrenia or any psychotic disorder, bipolar disorder, mental retardation, or cluster B personality disorders, mood disorder with postpartum onset, somatoform disorders, chronic fatigue syndrome, or fibromyalgia.
4. Meeting DSM-5 criteria for substance abuse (alcohol or drugs) within 12 months prior to Screening Visit or substance dependence (except nicotine and caffeine) within 6 months prior to the Screening Visit or positive test for drugs of abuse.
5. Moderate or high risk of violent behavior or suicide as assessed by any history of suicide attempt, or a score of > 4 on MADRS item 10.
6. History of inadequate treatment response on 2 or more occasions of treatment with approved antidepressants (paroxetine included) as defined by failure to improve when treated with a licensed dose for an adequate duration.
7. History of treatment with electroconvulsive therapy (ECT), transcranial magnetic stimulation (TMS), vagus nerve stimulation (VNS), or any related neuromodulator therapy within 6 months prior to the Screening Visit, including light therapy.
8. Subjects who, in the opinion of the investigator, should not discontinue, or participate in washout of a prohibited concomitant medication.
9. Receiving psychological treatments (e.g., cognitive behavior therapy, interpersonal psychotherapy, or psychodynamic psychotherapy) other than psychoeducational within 1 week prior to Baseline Visit (randomization) that has not remained constant for at least 3 months, or that may change during this study.
10. Significant past or present metabolic, hepatic (including > 3X upper limit of normal [ULN] in liver function test at Screening and Baseline), renal, hematological, pulmonary, cardiovascular, metabolic, gastrointestinal, or urological disorder.
11. Any history of drug or other significant allergy or known hypersensitivity to any of the study drugs.
12. History of malignant disease within the past 5 years with the exception of minor superficial skin diseases (i.e., basal cell carcinoma and squamous cell carcinoma).
13. Any medical condition that can potentially alter oral enteral absorption (e.g., gastrectomy), metabolism (e.g., liver failure), or excretion (e.g., renal failure) of the study drug.
14. Hypothyroidism or hyperthyroidism, unless stabilized by appropriate medication for at least 3 months prior to Screening (a normal thyroid-stimulating hormone [TSH] is required at Screening).
15. Pregnant or breast-feeding female.
16. Experimental drug use or experimental medical device use within 6 months before the planned start of treatment (Day 1).
17. Positive hepatitis B surface antigen (HBsAg), or hepatitis C antibody, or human immunodeficiency virus (HIV) 1 and 2 antibodies at Screening.
18. Subjects with a clinically significant electrocardiogram (ECG) abnormality at Screening or Baseline visits that could be a safety issue in the study, including QT interval value corrected for heart rate using the Fridericia's formula (QTcF) > 430 msec for males and > 450 msec for females.
19. Employees of the investigator or study center, when the employee has direct involvement in the proposed study or other studies under the direction of that investigator or study center; also family members of the employee or the investigator.
20. No legal capacity or limited legal capacity or unable to give an informed consent.
21. Subjects unlikely to cooperate in the study, and/or poor compliance anticipated by the investigator.

A summary of the demographic characteristics of the patients assigned to each arm of the study is shown in Table 2 below.

**Table 2: Demography**

| Demographic/Baseline Characteristics Statistic/Category | | | MIN-117 | | | Paroxetine 2C mg (N = 20) |
|---|---|---|---|---|---|---|
| | | Placebo (N = 20) | 0.5 mg (N = 21) | 2.5 mg (N = 21) | Total (N = 42) | |
| Age (years) | | | | | | |
| | n | 20 | 21 | 21 | 42 | 20 |
| | Mean | 48.4 | 50.0 | 46.3 | 48.2 | 48.5 |
| | SD (SE) | 13.3 (3.0) | 11.9 (2.6) | 13.0 (2.8) | 12.5 (1.9) | 11.5 (2.6) |
| | Median | 52.5 | 53.0 | 51.0 | 52.0 | 48.0 |
| | Min, Max | 24, 63 | 26, 66 | 23, 64 | 23, 66 | 29, 65 |
| Sex | | | | | | |
| | Male | 7 ( 35.0%) | 5 ( 23.8%) | 9 ( 42.9%) | 14 ( 33.3%) | 6 ( 30.0%) |
| | Female | 13 ( 65.0%) | 16 ( 76.2%) | 12 ( 57.1%) | 28 ( 66.7%) | 14 ( 70.0%) |
| Race | | | | | | |
| | Caucasian | 20 (100.0%) | 21 (100.0%) | 21 (100.0%) | 42 (100.0%) | 20 (100.0%) |
| Height (cm) | | | | | | |
| | n | 20 | 21 | 21 | 42 | 20 |
| | Mean | 167.7 | 166.4 | 170.0 | 168.2 | 168.8 |
| | SD (SE) | 8.7 (2.0) | 9.0 (2.0) | 7.8 (1.7) | 8.5 (1.3) | 8.7 (1.9) |
| | Median | 168.5 | 165.0 | 170.0 | 169.0 | 167.0 |
| | Min, Max | 155, 186 | 152, 182 | 156, 193 | 152, 193 | 157, 184 |
| Weight (kg) | | | | | | |
| | n | 20 | 21 | 21 | 42 | 20 |
| | Mean | 69.81 | 73.51 | 73.18 | 73.34 | 71.85 |
| | SD (SE) | 13.99 (3.13) | 16.82 (3.67) | 13.28 (2.90) | 14.97 (2.31) | 12.48 (2.79) |
| | Median | 68.55 | 70.00 | 71.00 | 70.75 | 71.75 |
| | Min, Max | 45, 95 | 48.7, 109.5 | 55.3, 100 | 48.7, 109.5 | 51, 105.2 |
| BMI (kg/m^2) | | | | | | |
| | n | 20 | 21 | 21 | 42 | 20 |
| | Mean | 24.7537 | 26.5076 | 25.4470 | 25.9773 | 24.9413 |
| | SD (SE) | 4.0616 (0.9082) | 5.2655 (1.1490) | 4.1434 (0.9042) | 4.7103 (0.7268) | 2.9164 (0.6521 |
| | Median | 24.3445 | 25.7810 | 24.8920 | 25.2585 | 24.4600 |
| | Min, Max | 18.73, 33.874 | 18.71, 33.949 | 19.563, 33.802 | 13.71, 33.949 | 20.69, 31.644 |

The change in HAM-A scores from baseline at weeks 1, 2, 4 and 6 weeks of treatment with placebo or a study drug are shown in Figure 1. As shown in Figure 1, each of the study drugs exhibited a reduction in the severity of anxiety symptoms compared to placebo in the intent to treat population (ITT) as measured by HAM-A scale over the course of the study, with patients treated with the 0.5 mg dose of MIN-117 exhibiting the fastest onset of this beneficial effect (compare Week 1 vs. Week 2 for all four arms). By Week 6, both doses of MIN-117 appeared to be comparable in terms of their ability to reduce the severity of the symptoms. Treatment with MIN-117 at both doses was well tolerated.

Although the study was not powered to achieve statistical significance, these results indicate that MIN-117 is capable of reducing the severity of one or more anxiety symptoms at total daily doses of 0.5 mg or 2.5 mg, with the 0.5 dose achieving a beneficial result over placebo as early as 2 weeks after starting therapy.

### EQUIVALENTS AND INCORPORATION BY REFERENCE

Unless otherwise defined, all technical and scientific terms used herein have the same meaning as commonly understood by one of ordinary skill in the art to which the invention belongs. In the specification and claims, the singular forms also include the plural unless the context clearly dictates otherwise.

It is to be understood that at least some of the descriptions of the invention have been simplified to focus on elements that are relevant for a clear understanding of the invention, while eliminating, for purposes of clarity, other elements that those of ordinary skill in the art will appreciate may also comprise a portion of the invention. However, because such elements are well known in the art, and because they do not necessarily facilitate a better understanding of the invention, a description of such elements is not provided herein.

Further, to the extent that the method does not rely on the particular order of steps set forth herein, the particular order of the steps should not be construed as limitation on the claims. The claims directed to the method of the present disclosure should not be limited to the performance of their steps in the order written, and one skilled in the art can readily appreciate that the steps may be varied and still remain within the spirit and scope of the present disclosure.

## Claims

1. A compound of formula I (Compound I), 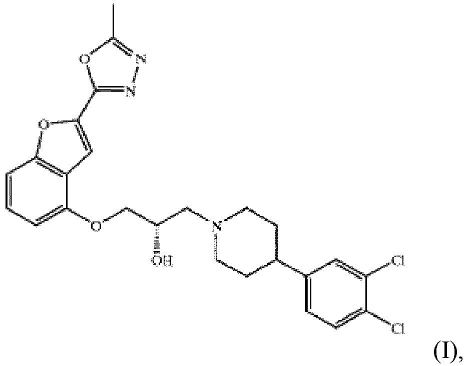 or a compound of formula II (Compound II) 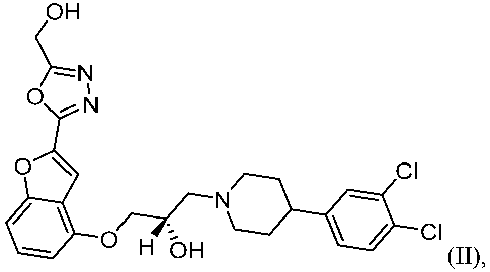 or a pharmaceutically acceptable salt, hydrate, or solvate of Compound I or II, for use in a method of treating at least one symptom of an anxiety disorder in a human subject who is diagnosed with both the anxiety disorder and a depression-related mood disorder, wherein the method comprises orally administering to the subject a total daily dose of Compound I or Compound II selected from the group consisting of:
(i) about 0.1 mg to less than 3.0 mg;
(ii) about 0.2 mg to about 2.9 mg;
(iii) about 0.3 mg to about 2.8 mg;
(iv) about 0.4 mg to about 2.7 mg;
(v) about 0.5 mg to about 2.6 mg;
(vi) about 0.6 mg to about 2.5 mg,
(vii) about 0.4 mg to about 0.6 mg; and
(viii) about 0.5 mg;
wherein the at least one symptom is a cognitive symptom of anxiety selected from the group consisting of: concentration problems, memory difficulties, and depressive symptoms.

2. The compound for use of claim 1, wherein the method comprises orally administering to the subject a total daily dose of Compound I or Compound II selected from the group consisting of:
(i) about 0.25 mg to about 2.5 mg;
(ii) about 0.25 mg to about 1.5 mg;
(iii) about 0.25 to about 1.0 mg;
(iv) about 0.25 mg to about 0.75 mg;
(v) about 0.1 mg to about 1.0 mg;
(vi) about 0.1 mg to about 0.75 mg;
(vii) about 0.1 mg to about 0.5 mg; and
(viii) about 0.5 mg.

3. The compound for use of claim 1 or 2, wherein the depression-related mood disorder is Major Depressive Disorder.

4. The compound for use of any one of claims 1 to 3, wherein the anxiety disorder is selected from the group consisting of: Separation Anxiety Disorder, Selective Mutism, Specific Phobia, Social Anxiety Disorder (Social Phobia), Panic Disorder, Panic Attack Specifier, Agoraphobia, Generalized Anxiety Disorder, Substance/Medication-Induced Anxiety Disorder, Anxiety Disorder Due to Another Medical Condition, Other Specified Anxiety Disorder, Unspecified Anxiety Disorder, Obsessive-Compulsive Disorder, Body Dysmorphic Disorder, Hoarding Disorder, Trichotillomania (Hair-Pulling Disorder), Excoriation (Skin-Picking) Disorder, Substance/Medication-Induced Obsessive-Compulsive and Related Disorder, Obsessive-Compulsive and Related Disorder Due to Another Medical Condition, Other Specified Obsessive-Compulsive and Related Disorder, Unspecified Obsessive-Compulsive and Related Disorder, Reactive Attachment Disorder, Disinhibited Social Engagement Disorder, Posttraumatic Stress Disorder, Acute Stress Disorder, Adjustment Disorders, Other Specified Trauma- and Stressor-Related Disorder, Unspecified Trauma- and Stressor-Related Disorder.

5. The compound for use of any one of claims 1 to 4, wherein Compound I is administered for a first treatment period selected from the group consisting of at least 1 week, at least 2 weeks, at least 3 weeks, at least 4 weeks, at least 5 weeks and at least 6 weeks and, if the first treatment period results in reduction of the at least one anxiety symptom in the subject, then the treating is continued for a second treatment period selected from the group consisting of at least 12 weeks, at least 24 weeks, and at least 48 weeks.

6. The compound for use of claim 1, wherein the compound is Compound I monohydrochloride and wherein the method comprises orally administering to the subject a total daily dose of between 0.5 mg to 2.5 mg of Compound I.

7. A composition comprising a compound of formula I (Compound I) 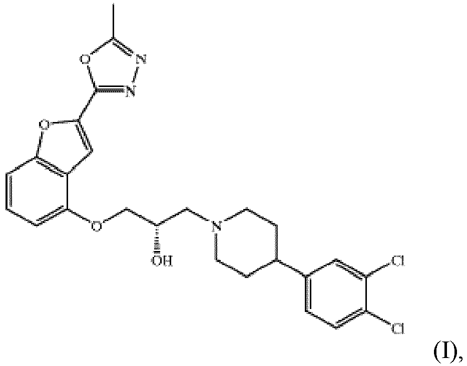 or a compound of formula II (Compound II) 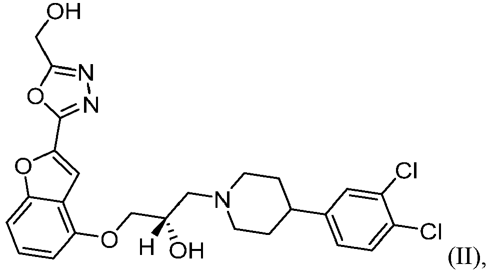 or a pharmaceutically acceptable salt, hydrate, or solvate of Compound I or Compound II, for use in a method for treating at least one symptom of an anxiety disorder in a human subject who is diagnosed with both the anxiety disorder and a depression-related mood disorder, wherein the method comprises orally administering to the subject a therapeutically effective amount of the composition, wherein the therapeutically effective amount is a total daily dose of Compound I or Compound II selected from the group consisting of:
(i) about 0.1 mg to less than 3.0 mg;
(ii) about 0.2 mg to about 2.9 mg;
(iii) about 0.3 mg to about 2.8 mg;
(iv) about 0.4 mg to about 2.7 mg;
(v) about 0.5 mg to about 2.6 mg;
(vi) about 0.6 mg to about 2.5 mg,
(vii) about 0.4 mg to about 0.6 mg; and
(viii) about 0.5 mg;
wherein the at least one symptom is a cognitive symptom of anxiety selected from the group consisting of: concentration problems, memory difficulties, and depressive symptoms.

8. The composition for use of claim 7, wherein the therapeutically effective amount is a total daily dose of Compound I or Compound II selected from the group consisting of:
(i) about 0.25 mg to about 2.5 mg;
(ii) about 0.25 mg to about 1.5 mg;
(iii) about 0.25 to about 1.0 mg;
(iv) about 0.25 mg to about 0.75 mg;
(v) about 0.1 mg to about 2.0 mg;
(vi) about 0.1 mg to about 1.0 mg; and
(vii) about 0.1 mg to about 0.5 mg.

9. The composition for use of claim 7 or 8, which comprises about 0.5 mg of Compound I or Compound II.

10. The composition for use of any one of claims 7 to 9, wherein the composition comprises (2S)-1-[4-(3,4-dichlorophenyl)piperidin-1-yl]-3-[2-(5-methyl-1,3,4-oxadiazol-2-yl)benzo[*b*]furan-4-yloxy]propan-2-ol monohydrochloride or (2*S*)-1-[4-(3,4-dichlorophenyl)piperidin-1-yl]-3-[2-(5-hydroxymethyl-1,3,4-oxadiazol-2-yl)benzo[*b*]furan-4-yloxy]propan-2-ol monohydrochloride.

11. The composition for use of any one of claims 7 to 9, wherein the composition comprises 0.5 mg of (2S)-1-[4-(3,4-dichlorophenyl)piperidin-1-yl]-3-[2-(5-methyl-1,3,4-oxadiazol-2-yl)benzo[*b*]furan-4-yloxy]propan-2-ol monohydrochloride.

12. The composition for use of any one of claims 7 to 11, wherein the depression-related mood disorder is selected from the group consisting of major depressive disorder, persistent depressive disorder, depression associated with a bipolar disorder, dysthymic disorder, cyclothymic disorder, and substance-induced mood disorder.

13. The composition for use of claim 12, wherein the depression-related mood disorder is major depressive disorder.

14. The composition for use of any one of claims 7 to 13, wherein the anxiety disorder is selected from the group consisting of: Separation Anxiety Disorder, Selective Mutism, Specific Phobia, Social Anxiety Disorder (Social Phobia), Panic Disorder, Panic Attack Specifier, Agoraphobia, Generalized Anxiety Disorder, Substance/Medication-Induced Anxiety Disorder, Anxiety Disorder Due to Another Medical Condition, Other Specified Anxiety Disorder, Unspecified Anxiety Disorder, Obsessive-Compulsive Disorder, Body Dysmorphic Disorder, Hoarding Disorder, Trichotillomania (Hair-Pulling Disorder), Excoriation (Skin-Picking) Disorder, Substance/Medication-Induced Obsessive-Compulsive and Related Disorder, Obsessive-Compulsive and Related Disorder Due to Another Medical Condition, Other Specified Obsessive-Compulsive and Related Disorder, Unspecified Obsessive-Compulsive and Related Disorder, Reactive Attachment Disorder, Disinhibited Social Engagement Disorder, Posttraumatic Stress Disorder, Acute Stress Disorder, Adjustment Disorders, Other Specified Trauma- and Stressor-Related Disorder, Unspecified Trauma- and Stressor-Related Disorder.

15. The composition for use of claim 7, wherein the compound is Compound I monohydrochloride and wherein the method comprises orally administering to the subject a therapeutically effective amount of the composition, wherein the therapeutically effective amount is a total daily dose of between 0.5 mg to 2.5 mg of Compound I.

16. A monohydrochloride salt of a compound of formula I (Compound I), 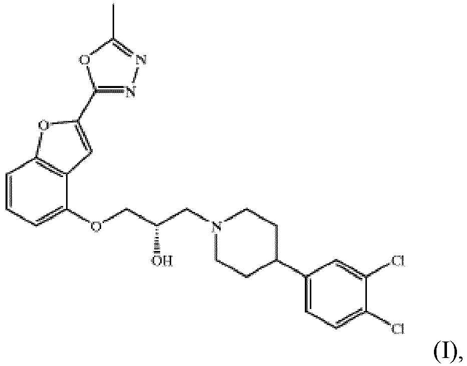 for use in a method of reducing symptoms of anxiety in an adult human subject, wherein the subject has been diagnosed with Major Depressive Disorder.

## Patentansprüche

1. Verbindung der Formel I (Verbindung I), 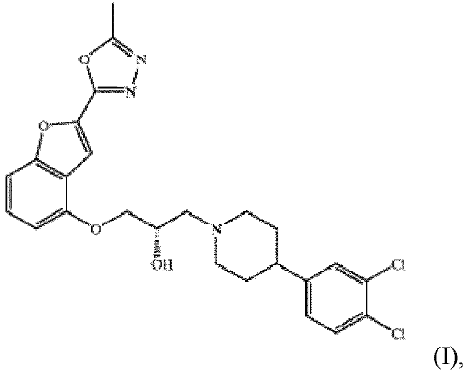 oder Verbindung der Formel II (Verbindung II) 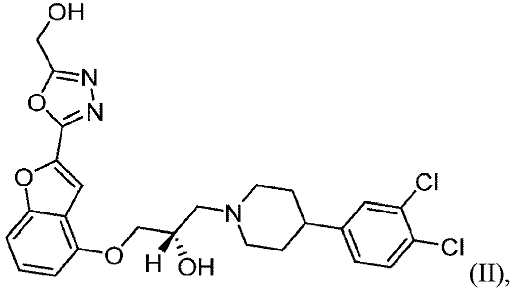 oder pharmazeutisch unbedenkliches Salz, Hydrat oder Solvat der Verbindung I oder II zur Verwendung in einem Verfahren zur Behandlung mindestens eines Symptoms einer Angststörung in einem humanen Subjekt, bei dem sowohl eine Angststörung als auch eine depressionsbedingte Gemütserkrankung diagnostiziert wurde, wobei das Verfahren die orale Verabreichung einer Gesamttagesdosis von Verbindung I oder Verbindung II ausgewählt aus der Gruppe bestehend aus:
(i) etwa 0,1 mg bis weniger als 3,0 mg,
(ii) etwa 0,2 mg bis etwa 2,9 mg,
(iii) etwa 0,3 mg bis etwa 2,8 mg,
(iv) etwa 0,4 mg bis etwa 2,7 mg,
(v) etwa 0,5 mg bis etwa 2,6 mg,
(vi) etwa 0,6 mg bis etwa 2,5 mg,
(vii) 0.4 mg bis etwa 0,6 mg und
(viii) etwa 0,5 mg
an das Subjekt umfasst, wobei es sich bei dem mindestens einen Symptom um ein kognitives Symptom von Angst ausgewählt aus der Gruppe bestehend aus Konzentrationsproblemen, Gedächtnisstörungen und depressiven Symptomen handelt.

2. Verbindung zur Verwendung nach Anspruch 1, wobei das Verfahren die orale Verabreichung einer Gesamttagesdosis von Verbindung I oder Verbindung II ausgewählt aus der Gruppe bestehend aus:
(i) etwa 0,25 mg bis etwa 2,5 mg,
(ii) etwa 0,25 mg bis etwa 1,5 mg,
(iii) etwa 0,25 bis etwa 1,0 mg,
(iv) etwa 0,25 mg bis etwa 0,75 mg,
(v) etwa 0,1 mg bis etwa 1,0 mg,
(vi) etwa 0,1 mg bis etwa 0,75 mg,
(vii) etwa 0,1 mg bis etwa 0,5 mg und
(viii) etwa 0,5 mg
an das Subjekt umfasst.

3. Verbindung zur Verwendung nach Anspruch 1 oder 2, wobei es sich bei der depressionsbedingten Gemütserkrankung um schwere Depression handelt.

4. Verbindung zur Verwendung nach einem der Ansprüche 1 bis 3, wobei die Angststörung ausgewählt ist aus der Gruppe bestehend aus: Trennungsangst, selektivem Mutismus, spezifischer Phobie, sozialer Angststörung (sozialer Phobie), Panikstörung, Panikattacken-Spezifikation, Agoraphobie, generalisierter Angststörung, substanz-/medikamenteninduzierter Angststörung, Angststörung aufgrund eines anderen medizinischen Leidens, anderen spezifizierten Angststörungen, nicht spezifizierter Angststörung, Zwangsstörung, körperdysmorpher Störung, Hortungsstörung, Trichotillomanie (Haarzupfstörung), Exkoriationsstörung (Hautausreißen), substanz-/medikamenteninduzierter Zwangsstörung und verwandten Störungen, Zwangsstörung und verwandten Störungen aufgrund eines anderen medizinischen Leidens, anderen spezifizierten Zwangsstörung und verwandten Störungen, nicht spezifizierter Zwangsstörung und verwandten Störungen, reaktiver Bindungsstörung, enthemmter sozialer Bindungsstörung, posttraumatischer Belastungsstörung, akuter Belastungsstörung, Anpassungsstörungen, anderen spezifizierten trauma- und belastungsbedingten Störungen, nicht spezifizierter trauma- und belastungsbedingter Störung.

5. Verbindung zur Verwendung nach einem der Ansprüche 1 bis 4, wobei Verbindung I über eine erste Behandlungsperiode ausgewählt aus der Gruppe bestehend aus mindestens 1 Woche, mindestens 2 Wochen, mindestens 3 Wochen, mindestens 4 Wochen, mindestens 5 Wochen und mindestens 6 Wochen verabreicht wird und, wenn die erste Behandlungsperiode zu einer Verminderung mindestens eines der Angstsymptome in dem Subjekt führt, die Behandlung über eine zweite Behandlungsperiode ausgewählt aus der Gruppe bestehend aus mindestens 12 Wochen, mindestens 24 Wochen und mindestens 48 Wochen fortgesetzt wird.

6. Verbindung zur Verwendung nach Anspruch 1, wobei es sich bei der Verbindung um Verbindung-I-monohydrochlorid handelt und wobei das Verfahren die orale Verabreichung einer Gesamttagesdosis von 0,5 mg bis 2,5 mg Verbindung I an das Subjekt umfasst.

7. Zusammensetzung, umfassend eine Verbindung der Formel I (Verbindung I) 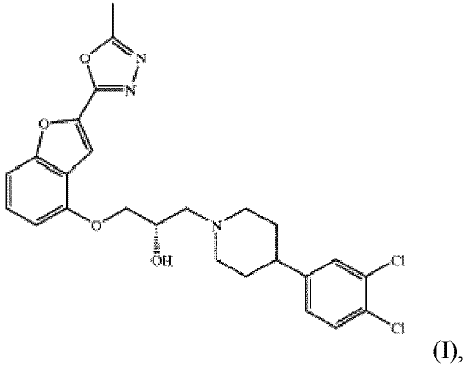 oder eine Verbindung der Formel II (Verbindung II) 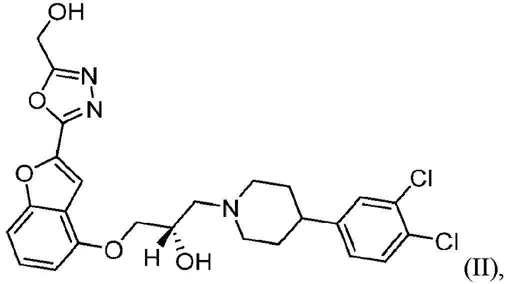 oder ein pharmazeutisch unbedenkliches Salz, Hydrat oder Solvat der Verbindung I oder II zur Verwendung in einem Verfahren zur Behandlung mindestens eines Symptoms einer Angststörung in einem humanen Subjekt, bei dem sowohl eine Angststörung als auch eine depressionsbedingte Gemütserkrankung diagnostiziert wurde, wobei das Verfahren die orale Verabreichung einer therapeutisch wirksamen Menge der Zusammensetzung an das Subjekt umfasst, wobei es sich bei der therapeutisch wirksamen Menge um eine Gesamttagesdosis von Verbindung I oder Verbindung II ausgewählt aus der Gruppe bestehend aus:
(i) etwa 0,1 mg bis weniger als 3,0 mg,
(ii) etwa 0,2 mg bis etwa 2,9 mg,
(iii) etwa 0,3 mg bis etwa 2,8 mg,
(iv) etwa 0,4 mg bis etwa 2,7 mg,
(v) etwa 0,5 mg bis etwa 2,6 mg,
(vi) etwa 0,6 mg bis etwa 2,5 mg,
(vii) 0.4 mg bis etwa 0,6 mg und
(viii) etwa 0,5 mg,
handelt, wobei es sich bei dem mindestens einen Symptom um ein kognitives Symptom von Angst ausgewählt aus der Gruppe bestehend aus Konzentrationsproblemen, Gedächtnisstörungen und depressiven Symptomen handelt.

8. Zusammensetzung zur Verwendung nach Anspruch 7, wobei es sich bei der therapeutisch wirksamen Menge um eine Gesamttagesdosis von Verbindung I oder Verbindung II ausgewählt aus der Gruppe bestehend aus:
(i) etwa 0,25 mg bis etwa 2,5 mg,
(ii) etwa 0,25 mg bis etwa 1,5 mg,
(iii) etwa 0,25 bis etwa 1,0 mg,
(iv) etwa 0,25 mg bis etwa 0,75 mg,
(v) etwa 0,1 mg bis etwa 2,0 mg,
(vi) etwa 0,1 mg bis etwa 1,0 mg und
(vii) etwa 0,1 mg bis etwa 0,5 mg
handelt.

9. Zusammensetzung zur Verwendung nach Anspruch 7 oder 8, welche etwa 0,5 mg Verbindung I oder Verbindung II umfasst.

10. Zusammensetzung zur Verwendung nach einem der Ansprüche 7 bis 9, wobei die Zusammensetzung (2S)-1-[4-(3,4-Dichlorphenyl)piperidin-1-yl]-3-[2-(5-methyl-1,3,4-oxadiazol-2-yl)benzo[*b*]furan-4-yloxy]propan-2-ol-monohydrochlorid oder (2S)-1-[4-(3,4-Dichlorphenyl)piperidin-1-yl]-3-[2-(5-hydroxymethyl-1,3,4-oxadiazol-2-yl)benzo[*b*]furan-4-yloxy]propan-2-ol-monohydrochlorid umfasst.

11. Zusammensetzung zur Verwendung nach einem der Ansprüche 7 bis 9, wobei die Zusammensetzung 0,5 mg (2S)-1-[4-(3,4-Dichlorphenyl)piperidin-1-yl]-3-[2-(5-methyl-1,3,4-oxadiazol-2-yl)benzo[*b*]furan-4-yloxy]propan-2-ol-monohydrochlorid umfasst.

12. Zusammensetzung zur Verwendung nach einem der Ansprüche 7 bis 11, wobei die depressionsbedingte Gemütserkrankung aus der aus schwerer Depression, anhaltender depressiver Störung, mit einer bipolaren Störung assoziierter Depression, dysthymischer Störung, zyklothymischer Störung und substanzinduzierten Gemütserkrankung bestehenden Gruppe ausgewählt ist.

13. Zusammensetzung zur Verwendung nach Anspruch 12, wobei es sich bei der depressionsbedingten Gemütserkrankung um schwere Depression handelt.

14. Zusammensetzung zur Verwendung nach einem der Ansprüche 7 bis 13, , wobei die Angststörung ausgewählt ist aus der Gruppe bestehend aus: Trennungsangst, selektivem Mutismus, spezifischer Phobie, sozialer Angststörung (sozialer Phobie), Panikstörung, Panikattacken-Spezifikation, Agoraphobie, generalisierter Angststörung, substanz-/medikamenteninduzierter Angststörung, Angststörung aufgrund eines anderen medizinischen Leidens, anderen spezifizierten Angststörungen, nicht spezifizierter Angststörung, Zwangsstörung, körperdysmorpher Störung, Hortungsstörung, Trichotillomanie (Haarzupfstörung), Exkoriationsstörung (Hautausreißen), substanz-/medikamenteninduzierter Zwangsstörung und verwandten Störungen, Zwangsstörung und verwandten Störungen aufgrund eines anderen medizinischen Leidens, anderen spezifizierten Zwangsstörung und verwandten Störungen, nicht spezifizierter Zwangsstörung und verwandten Störungen, reaktiver Bindungsstörung, enthemmter sozialer Bindungsstörung, posttraumatischer Belastungsstörung, akuter Belastungsstörung, Anpassungsstörungen, anderen spezifizierten trauma- und belastungsbedingten Störungen, nicht spezifizierter trauma- und belastungsbedingter Störung.

15. Zusammensetzung zur Verwendung nach Anspruch 7, , wobei es sich bei der Verbindung um Verbindung-I-monohydrochlorid handelt und wobei das Verfahren die orale Verabreichung einer therapeutisch wirksamen Menge der Zusammensetzung an das Subjekt umfasst, wobei es sich bei der therapeutisch wirksamen Menge um eine Gesamttagesdosis von 0,5 mg bis 2,5 mg Verbindung I handelt.

16. Monohydrochloridsalz einer Verbindung der Formel I (Verbindung I), 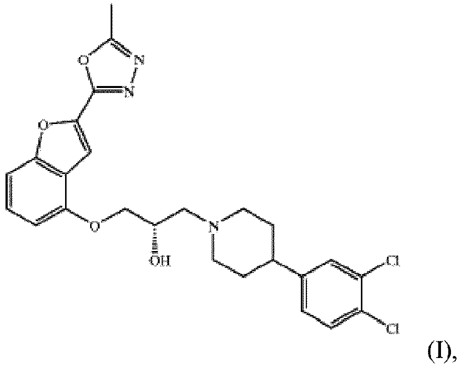 zur Verwendung in einem Verfahren zur Verminderung der Symptome von Angst in einem erwachsenen humanen Subjekt, wobei bei dem Subjekt eine schwere Depression diagnostiziert wurde.

## Revendications

1. Composé de formule I (composé I), 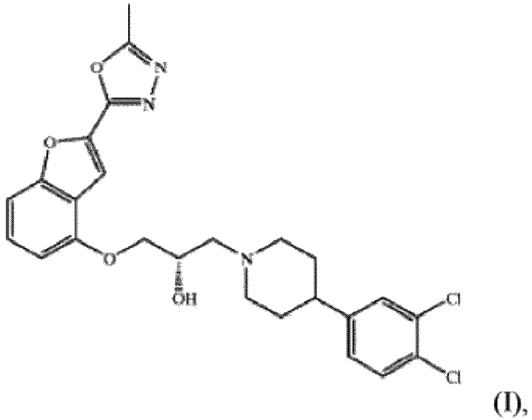 ou composé de formule II (composé II) 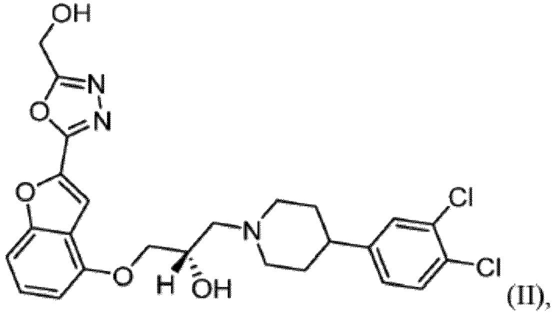 ou sel, hydrate, ou solvate pharmaceutiquement acceptable du composé I ou II, pour une utilisation dans un procédé de traitement d'au moins un symptôme d'un trouble anxieux chez un sujet humain qui a été diagnostiqué à la fois du trouble anxieux et d'un trouble de l'humeur lié à une dépression, le procédé comprenant une administration par voie orale au sujet d'une dose quotidienne totale de composé I ou de composé II choisie dans le groupe constitué par :
(i) environ 0,1 mg à moins de 3,0 mg ;
(ii) environ 0,2 mg à environ 2,9 mg ;
(iii) environ 0,3 mg à environ 2,8 mg ;
(iv) environ 0,4 mg à environ 2,7 mg ;
(v) environ 0,5 mg à environ 2,6 mg ;
(vi) environ 0,6 mg à environ 2,5 mg ;
(vii) environ 0,4 mg à environ 0,6 mg ; et
(viii) environ 0,5 mg ;
l'au moins un symptôme étant un symptôme cognitif d'anxiété choisi dans le groupe constitué par : des problèmes de concentration, des difficultés de mémoire, et des symptômes dépressifs.

2. Composé pour une utilisation selon la revendication 1, le procédé comprenant une administration par voie orale au sujet d'une dose quotidienne totale de composé I ou de composé II choisie dans le groupe constitué par :
(i) environ 0,25 mg à environ 2,5 mg ;
(ii) environ 0,25 mg à environ 1,5 mg ;
(iii) environ 0,25 mg à environ 1,0 mg ;
(iv) environ 0,25 mg à environ 0,75 mg ;
(v) environ 0,1 mg à environ 1,0 mg ;
(vi) environ 0,1 mg à environ 0,75 mg ;
(vii) environ 0,1 mg à environ 0,5 mg ; et
(viii) environ 0,5 mg.

3. Composé pour une utilisation selon la revendication 1 ou 2, le trouble de l'humeur lié à une dépression étant un trouble dépressif majeur.

4. Composé pour une utilisation selon l'une quelconque des revendications 1 à 3, le trouble anxieux étant choisi dans le groupe constitué par : trouble anxieux de séparation, mutisme sélectif, phobie spécifique, trouble anxieux social (phobie sociale), trouble panique, spécification de l'attaque de panique, agoraphobie, trouble anxieux généralisé, trouble anxieux induit par une substance/un médicament, trouble anxieux dû à une autre affection médicale, autre trouble anxieux spécifié, trouble anxieux non spécifié, trouble obsessionnel-compulsif, trouble dysmorphique du corps, trouble de la thésaurisation, trichotillomanie (trouble de l'arrachage des cheveux), trouble de l'excoriation (arrachage de la peau), trouble obsessionnel-compulsif et apparenté induit par une substance ou un médicament, trouble obsessionnel-compulsif et apparenté dû à une autre affection médicale, autre trouble obsessionnel-compulsif et apparenté spécifié, trouble obsessionnel-compulsif et apparenté non spécifié, trouble de l'attachement réactif, trouble de l'engagement social désinhibé, trouble du stress post-traumatique, trouble du stress aigu, troubles de l'adaptation, autre trouble spécifique lié à un traumatisme ou à un facteur de stress, trouble non spécifié lié à un traumatisme ou à un facteur de stress.

5. Composé pour une utilisation selon l'une quelconque des revendications 1 à 4, le composé I étant administré pour une première période de traitement choisie dans le groupe constitué par au moins une semaine, au moins 2 semaines, au moins 3 semaines, au moins 4 semaines, au moins 5 semaines et au moins 6 semaines et, si la première période de traitement entraîne une réduction de l'au moins un symptôme d'anxiété chez le sujet, alors le traitement est poursuivi pour une deuxième période de traitement choisie dans le groupe constitué par au moins 12 semaines, au moins 24 semaines, et au moins 48 semaines.

6. Composé pour une utilisation selon la revendication 1, le composé étant le monochlorhydrate du composé I et le procédé comprenant une administration par voie orale au sujet d'une dose quotidienne totale comprise entre 0,5 mg et 2,5 mg du composé I.

7. Composition comprenant un composé de formule I (composé I) 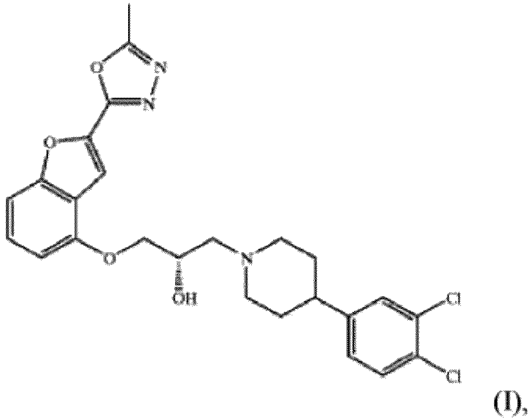 ou un composé de formule II (composé II) 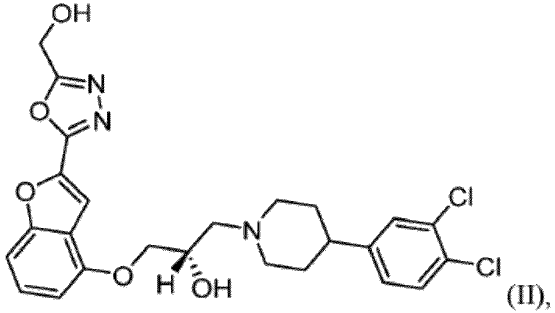 ou un sel, un hydrate, ou un solvate pharmaceutiquement acceptable du composé I ou II, pour une utilisation dans un procédé pour le traitement d'au moins un symptôme d'un trouble anxieux chez un sujet humain qui a été diagnostiqué à la fois du trouble anxieux et d'un trouble de l'humeur lié à une dépression, le procédé comprenant une administration par voie orale au sujet d'une dose quotidienne totale de composé I ou de composé II choisie dans le groupe constitué par :
(i) environ 0,1 mg à moins de 3,0 mg ;
(ii) environ 0,2 mg à environ 2,9 mg ;
(iii) environ 0,3 mg à environ 2,8 mg ;
(iv) environ 0,4 mg à environ 2,7 mg ;
(v) environ 0,5 mg à environ 2,6 mg ;
(vi) environ 0,6 mg à environ 2,5 mg ;
(vii) environ 0,4 mg à environ 0,6 mg ; et
(viii) environ 0,5 mg ;
l'au moins un symptôme étant un symptôme cognitif d'anxiété choisi dans le groupe constitué par : des problèmes de concentration des difficultés de mémoire, et des symptômes dépressifs.

8. Composition pour une utilisation selon la revendication 7, la quantité thérapeutiquement efficace étant une dose quotidienne totale de composé I ou de composé II choisie dans le groupe constitué par :
(i) environ 0,25 mg à environ 2,5 mg ;
(ii) environ 0,25 mg à environ 1,5 mg ;
(iii) environ 0,25 mg à environ 1,0 mg ;
(iv) environ 0,25 mg à environ 0,75 mg ;
(v) environ 0,1 mg à environ 2,0 mg ;
(vi) environ 0,1 mg à environ 1,0 mg ; et
(vii) environ 0,1 mg à environ 0,5 mg.

9. Composition pour une utilisation selon la revendication 7 ou 8, qui comprend environ 0,5 mg de composé I ou de composé II.

10. Composition pour une utilisation selon l'une quelconque des revendications 7 à 9 comprenant du monochlorhydrate de (2S)-1-[4-(3,4-dichlorophényl)pipéridin-1-yl]-3-[2-(5-méthyl-1,3,4-oxadiazol-2-yl)benzo[b]furan-4-yloxy]propan-2-ol ou du monochlorhydrate de (2S)-1-[4-(3,4-dichlorophényl)pipéridin-1-yl]-3-[2-(5-hydroxyméthyl-1,3,4-oxadiazol-2-yl)benzo[*b*]furan-4-yloxy]propan-2-ol.

11. Composition pour une utilisation selon l'une quelconque des revendications 7 à 9, la composition comprenant 0,5 mg de monochlorhydrate de (2S)-1-[4-(3,4-dichlorophényl)pipéridin-1-yl]-3-[2-(5-méthyl-1,3,4-oxadiazol-2-yl)benzo[b]furan-4-yloxy]propan-2-ol.

12. Composition pour une utilisation selon l'une quelconque des revendications 7 à 11, le trouble de l'humeur lié à une dépression étant choisi dans le groupe constitué par un trouble dépressif majeur, un trouble dépressif persistant, une dépression associée à un trouble bipolaire, un trouble dysthymique, un trouble cyclothymique, et un trouble de l'humeur induit par une substance.

13. Composition pour une utilisation selon la revendication 12, le trouble de l'humeur lié à une dépression étant un trouble dépressif majeur.

14. Composition pour une utilisation selon l'une quelconque des revendications 7 à 13, le trouble anxieux étant choisi dans le groupe constitué par : trouble anxieux de séparation, mutisme sélectif, phobie spécifique, trouble anxieux social (phobie sociale), trouble panique, spécification de l'attaque de panique, agoraphobie, trouble anxieux généralisé, trouble anxieux induit par une substance/un médicament, trouble anxieux dû à une autre affection médicale, autre trouble anxieux spécifié, trouble anxieux non spécifié, trouble obsessionnel-compulsif, trouble dysmorphique du corps, trouble de la thésaurisation, trichotillomanie (trouble de l'arrachage des cheveux), trouble de l'excoriation (arrachage de la peau), trouble obsessionnel-compulsif et apparenté induit par une substance ou un médicament, trouble obsessionnel-compulsif et apparenté dû à une autre affection médicale, autre trouble obsessionnel-compulsif et apparenté spécifié, trouble obsessionnel-compulsif et apparenté non spécifié, trouble de l'attachement réactif, trouble de l'engagement social désinhibé, trouble du stress post-traumatique, trouble du stress aigu, troubles de l'adaptation, autre trouble spécifique lié à un traumatisme ou à un facteur de stress, trouble non spécifié lié à un traumatisme ou à un facteur de stress.

15. Composition pour une utilisation selon la revendication 7, le composé étant le monochlorhydrate du composé I et, le procédé comprenant une administration par voie orale au sujet d'une quantité thérapeutiquement efficace de la composition, la quantité thérapeutiquement efficace étant une dose quotidienne totale comprise entre 0,5 mg et 2,5 mg de composé I.

16. Sel de monochlorhydrate d'un composé de formule I (composé I), 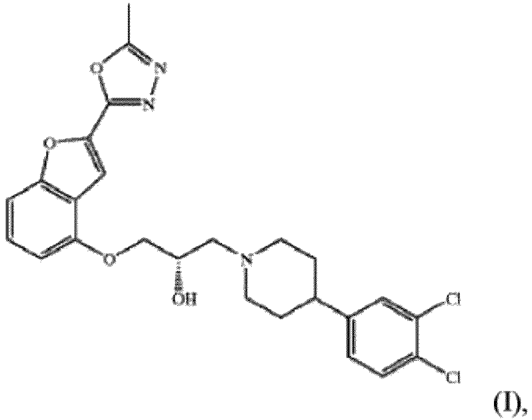 pour une utilisation dans un procédé de réduction de symptômes d'anxiété chez un sujet humain adulte, le sujet ayant été diagnostiqué d'un trouble dépressif majeur.
